# EUROPÄISCHE PATENTSCHRIFT

(11) **EP 1 240 919 B1**
(45) Veröffentlichungstag und Bekanntmachung des Hinweises auf die Patenterteilung: **24.08.2005**
(21) Anmeldenummer: 01127055.0
(22) Anmeldetag: 14.11.2001
(51) Int. Cl.: A61N 5/067

(54) **Laser-Behandlungsgerät**
Laser treatment apparatus
Appareil de traitement par laser

(30) Priorität: 15.02.2001 DE 20102681 U
(43) Veröffentlichungstag der Anmeldung: 18.09.2002
(73) Patentinhaber: petra-electric Peter Hohlfeldt GmbH & Co., 89331 Burgau (DE)
(72) Erfinder: Gierth, Rolf, 89331 Burgau (DE); Höhne, Joachim, 89331 Burgau (DE)
(74) Vertreter: Grünecker, Kinkeldey, Stockmair & Schwanhäusser Anwaltssozietät

(56) Entgegenhaltungen:
- EP-A- 0 320 080
- WO-A-97/35635
- DE-A- 3 226 507
- US-A- 5 150 704

## Beschreibung

Die vorliegende Erfindung bezieht sich auf ein Laser-Behandlungsgerät gemäß Oberbegriff des Anspruchs 1.

Aus der DE 32 26 507 A1 ist ein lichttechnisches Gerät für akupunkturähnliche, therapeutische Behandlung bekannt.

Ein weiteres Laser-Behandlungsgerät ist aus der EP 722 750 bekannt. Dort handelt es sich speziell um ein Laser-Akupunkturgerät. Diese Geräte werden auf die Haut gesetzt und ermöglichen ein punktuelles Bestrahlen der Haut. Um einen Akupunkturpunkt aufzufinden, ist das bekannte Laser-Akupunkturgerät mit einer Sucheinrichtung ausgestattet, die den Hautwiderstand misst. Die Anzeigeeinrichtung verfügt über ein Leuchtdiodenband. Zum Aufsuchen des Akupunkturpunktes nimmt die Bedienperson das Laser-Behandlungsgerät mit dem stiftförmigen Gehäuse in die Hand und bewegt eine Spitze des Geräts über die Hautoberfläche. Bei Annäherung an einen Akupunkturpunkt erfasst das Gerät die Veränderung des Hautwiderstandes und bringt eine oder mehrere der Leuchtdioden zum Leuchten, wobei die Annäherung an einen Akupunkturpunkt durch eine steigende Anzahl von leuchtenden Dioden dargestellt wird. Wenn der Akupunkturpunkt überstrichen wurde, nimmt die Anzahl der aufleuchtenden Dioden wieder ab, wodurch ein Entfernen vom Akupunkturpunkt feststellbar ist. Hat man nun einen Akupunkturpunkt lokalisiert, kann eine Bestrahlung des Akupunkturpunktes mit dem Laser beginnen. Um Verletzungen der Haut zu vermeiden, sind die Laser relativ leistungsschwach. Dennoch kann es vereinzelt zu Erwärmung und Hautveränderungen kommen. Aus gesundheitlichen Gründen kann daher die Bestrahlung nicht beliebig lange erfolgen.

Häufig kommt es jedoch ungewollt zu einer längeren Bestrahlungsdauer, die unter Umständen gesundheitsschädlich sein könnte.

Aufgabe der Erfindung ist es daher, ein Laser-Behandlungsgerät der eingangs genannten Art sicherer zu gestalten.

Die Aufgabe wird erfindungsgemäß gelöst durch ein Laser-Behandlungsgerät der eingangs genannten Art, bei dem durch Bedienen der Betätigungseinrichtung das Laser-Behandlungsgerät in Betrieb nehmbar ist.

Diese Lösung ist einfach und hat den Vorteil, dass das Laser-Behandlungsgerät durch ein und dieselbe Einrichtung eingeschaltet werden kann und sich unterschiedliche Behandlungszeiträume vorwählen lassen. Da das Gerät nach Erreichen der vorgewählten Zeit selbsttätig abschaltet, wird sichergestellt, dass die Behandlungszeiträume nicht zu lang sind. Dadurch erhöht sich zum einen der Bedienkomfort zum anderen die Betriebssicherheit. Ungewollt lange oder ungleichmäßige Behandlungszeiträume lassen sich durch das neuartige Laser-Behandlungsgerät wirkungsvoll vermeiden.

Eine sehr einfache Konstruktion ergibt sich, wenn die Betätigungseinrichtung einen vom Benutzer bedienbaren Betätigungsschalter aufweist.

Von Vorteil kann es dabei sein, wenn der Betätigungsschalter eine Taste ist. Dabei lässt sich eine besonders komfortable Bedienung des Laser-Behandlungsgeräts verwirklichen.

Als günstig kann es sich erweisen, wenn die Anzeigeeinrichtung mehrere Leuchtdioden aufweist. Dadurch lässt sich auf einfache Weise eine Anzeigeeinrichtung verwirklichen.

Dabei kann es sich als günstig erweisen, wenn die Leuchtdioden in einer Reihe im Gehäuse angeordnet sind. Dadurch lässt sich eine besondere übersichtliche Grafik der Anzeigeeinrichtung verwirklichen.

Als vorteilhaft kann es sich erweisen, wenn zumindest sechs Leuchtdioden vorgesehen sind. Mit sechs Leuchtdioden lässt sich für den vorgegebenen Anwendungszweck eine genügend große Anzeigegenauigkeit bei gleichzeitig geringem Aufwand realisieren.

Zur Vereinfachung der Bedienbarkeit des Laser-Behandlungsgeräts kann den Leuchtdioden benachbart wenigstens eine Skalierung am Gehäuse vorgesehen sein, wobei jeweils Skalenwerte einer der Leuchtdioden zugeordnet sind.

Als vorteilhaft können sich hierbei Skalenwerte in einem Bereich von 0 - 60 Sekunden erweisen. Wenn man sechs Leuchtdioden verwendet, kann dann Intervalle von 10 Sekunden jeweils eine Leuchtdiode zugeordnet werden. Eine Zeitspanne von 0 - 60 Sekunden eignet sich insbesondere zur Akupunkturbehandlung, wo die Bestrahlungszeiträume in diesem Zeitintervall liegen.

Alternativ kann es sich als günstig erweisen, wenn die Skalenwerte in einem Bereich von 0 - 12 Minuten liegen. Dieser Zeitraum eignet sich für Behandlungen bei Dauerbestrahlung. Es können den sechs Leuchtdioden Zahlenwerte von 2- 12 Minuten zugeordnet werden.

In einer vorteilhaften Weiterbildung kann das Laser-Behandlungsgerät durch Bedienen der Taste in Betrieb nehmbar sein. Die Taste kann somit sowohl die Funktion des Einschaltens und Ausschaltens des Laser-Behandlungsgeräts, als auch die Funktion des Festlegens der maximalen Behandlungszeit übernehmen.

Von Vorteil kann es sich dabei erweisen, wenn bei Betätigen der Taste zunächst die erste Leuchtdiode aufleuchtet, bei nochmaligem Betätigen der Taste zusätzlich die nächste folgende Leuchtdiode aufleuchtet und bei nochmaligem Betätigen nur noch die nachfolgende Leuchtdiode aufleuchtet. Man kann also somit durch Betätigen der Taste eine Leuchtdiode zum Aufleuchten bringen, die einem vorgegebenen Wert der Skalierung entspricht, wodurch die jeweilige Behandlungsdauer festgelegt wird. Wenn zwei Leuchtdioden nebeneinander leuchten, so kann dies auf einen Zwischenwert der Skalierung zwischen den beiden Skalierungswerten hinweisen. Wenn also die beiden Leuchtdioden leuchten, die den Zeitwerten 6 und 8 Minuten zugeordnet sind, so deutet dies auf den Zwischenwert hin, also 7 Minuten.

In einer vorteilhaften Weiterbildung der Erfindung kann zusätzlich eine Sucheinrichtung zum Ermitteln eines Akupunkturpunktes vorgesehen sein, wobei mit der Sucheinrichtung bei Annäherung an einen Akupunkturpunkt ein akustisches Signal ausgebbar ist. Diese Ausgabe eines akustischen Signals ist bedienungsfreundlicher als den bislang bekannten Akupunktur-Lasern, bei denen die Menge der aufleuchtenden Leuchtdioden auf die Nähe eines Akupunkturpunktes hinweist. Bei diesen Geräten ist es nämlich immer erforderlich, dass die Bedienperson die Anzahl der aufleuchtenden Leuchtdioden ständig visuell ermittelt. Bei einem akustischen Signal kann sich die Person besser auf das Suchen des Akupunkturpunktes konzentrieren. Die Sucheinrichtung kann auch unabhängig von der Betätigungseinrichtung zum Vorwählen eines Zeitraumes und der Anzeigeeinrichtung zum Darstellen der Information über den vorgewählten Zeitraum verwendet werden.

Hierbei kann es sich als vorteilhaft erweisen, wenn bei Annäherung an den Akupunkturpunkt die Frequenz des ausgegebenen Signals zunimmt. Dadurch lässt sich ein Akupunkturpunkt sehr genau ermitteln.

Weiterhin kann es sich als günstig erweisen, wenn das Laser-Behandlungsgerät eine Vorwahleinrichtung aufweist, mit der ein Akupunkturbetrieb oder ein Bestrahlungsbetrieb vorwählbar ist. Bei Akupunkturbetrieb sind die Betriebszeiten wesentlich kürzer. Hier kann die Behandlungszeit anhand der Skalen bis auf maximal 60 Sekunden eingestellt werden. Im Falle der Bestrahlungsbehandlung liegt die Behandlungsdauer bei maximal 12 Minuten. Entsprechend erfolgt das Vorwählen der Behandlungsdauer anhand der bis 12 Minuten reichenden Skala. Durch den Umschalter ist es möglich mit nur einer Reihe von Leuchtdioden eine Zeitvorwahl sowohl für den Akupunkturbetrieb als auch für den Bestrahlungsbetrieb vorzunehmen, wobei beide Skalen am Gehäuse angebracht sind.

Nachfolgend wird die Wirkungs- und Funktionsweise der Erfindung anhand eines Ausführungsbeispiels näher erläutert.

Es zeigen:
- Fig. 1: das erfindungsgemäße Laser-Behandlungsgerät in einer perspektivischen Darstellung;
- Fig. 2: eine schematische Darstellung der einzelnen Komponenten des Laser-Behandlungsgeräts.

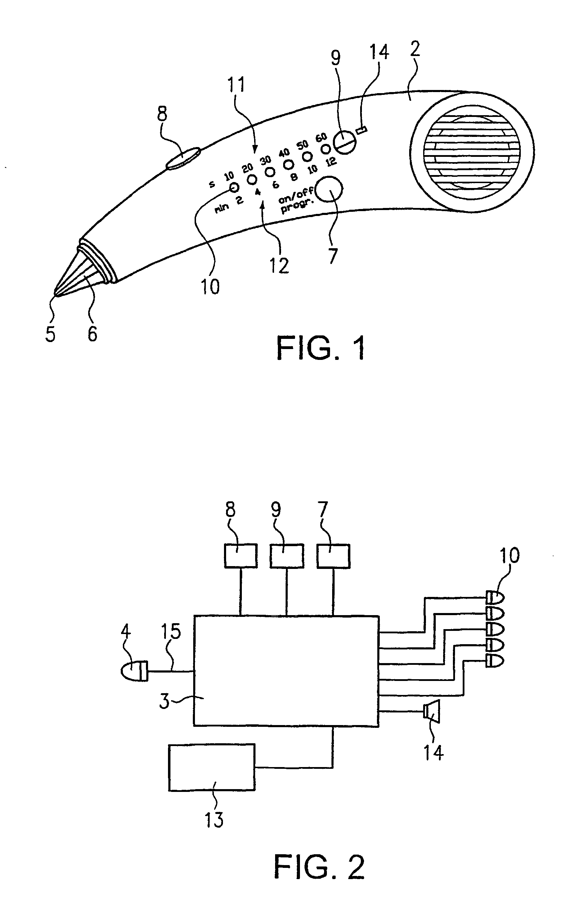

In Fig. 1 ist das Laser-Behandlungsgerät 1 in einer Schrägansicht dargestellt. Bei dem Laser-Behandlungsgerät handelt es sich um ein Laser-Akupunkturgerät. Das Laser-Behandlungsgerät 1 verfügt über ein Gehäuse 2 aus Kunststoff, in dem eine Steuerungseinrichtung 3 aufgenommen ist. Das Gehäuse 2 ist im Wesentlichen stiftförmig und kann in der Art eines Stiftes von einer Bedienperson geführt werden. Hierzu ist das Gehäuse 2 ergonomisch gerundet.

Eine Laserdiode 4 befindet sich im Innern des Gehäuses. Die Laserdiode liefert Rotlicht mit einer Wellenlänge zwischen 635 und 670 m. Die Leistung des Lasers beträgt weniger als 5 mW. Es handelt sich um eine Laser der Laser-Schutzklasse 3A. Bei derartigen Lasern wird der Schutz des Auges durch die Abwendungsreaktion und den Lidschutzreflex gewährleistet. Ein von der Laserdiode erzeugtes Licht tritt über eine Linse 5 an der Spitze 6 des Gehäuses aus. Die Spitze 6 besteht aus Metall und ist hartvergoldet um gegenüber MikroOrganismen abweisend und desinfizierbar zu sein.

Im Gehäuse 2 befinden sich Öffnungen, durch die ein Taster 7 und zwei Umschalter 8 und 9 von außen her für eine Bedienperson zugänglich sind. Die beiden Umschalter 8 und 9 sind von bekannter Bauart und können zwischen zwei Stellungen verschoben werden. In der Darstellung in Fig. 1 befindet sich der Umschalter 9 in einer oberen Stellung und der Schalter 8 in einer von der Spitze weg bewegten hinteren Stellung. Umschalter 9 kann in der Darstellung in Fig. 1 nach unten in eine zweite Stellung überführt werden und Schalter 8 in eine zweite näher zur Spitze verschobene Stellung.

Der Taster 7 ist ein Taster, der von einer Person mit dem Finger in das Gehäuse eindrückbar ist und in bekannter Weise unter Federvorspannung steht, so dass er bei Loslassen in die Fig. 1 dargestellte Ausgangsstellung zurückgedrückt wird.

Leuchtdioden 10 sind in einer Reihe angeordnet und durch Öffnungen im Gehäuse sichtbar. Oberhalb und unterhalb der Leuchtdioden befinden sich zwei Skalen 11 und 12. Skala 11 verfügt über die Werte 10, 20, 30, 40, 50 und 60, wobei jeder Zahlenwert jeweils einer Diode zugeordnet ist. Das kleine Symbol "s" weist daraufhin, dass es sich hierbei um die Angaben von Sekunden handeln soll.

Die Skala 12 verfügt über die Zahlenwerte 2, 4, 6, 8, 10 und 12 sowie den Hinweis "min", was auf Minuten hinweisen soll. Auch hier sind die Zahlenwerte jeweils einer Leuchtdiode zugeordnet. Insgesamt sind sechs Leuchtdioden vorhanden.

Als weitere Symbole befinden sich in der Darstellung rechts neben dem Umschalter 9 zwei übereinander angeordnete Zeichen 14, wobei jedes der Zeichen einer Stellung des Umschalters 9 zugeordnet ist.

Dem Taster 7 ist zudem noch die Beschriftung "on/off" sowie "prog" zugeordnet. Dies deutet daraufhin, dass der Taster 7 zum einen zum Einschalten des Gerätes dient und zum anderen zum Programmieren bzw. Vorwählen der maximalen Behandlungsdauer.

Die Stromversorgung des Laser-Behandlungsgeräts erfolgt durch Batterien 13. Diese sind ebenfalls im Gehäuse 2 des Akupunkturgeräts untergebracht. In bekannter Weise kann ein nicht dargestellter Deckel vorgesehen sein, um die Batterien auszutauschen.

Ein Tonerzeuger 14 ist mit der Steuerung verbunden und kann über den Umschalter 9 aktiviert werden.

In der Darstellung in Fig. 2 sind die einzelnen Komponenten des Laser-Behandlungsgeräts schematisch dargestellt, wobei die einzelnen Komponenten jeweils über symbolisch dargestellte Leitungen 15 mit der Steuerung 3 verbunden sind. Weiterhin verfügt das Laser-Behandlungsgerät über eine nicht näher dargestellte Gegenelektrode.

Die Wirkungs- und Funktionsweise des erfindungsgemäßen Laser-Behandlungsgeräts werden nachfolgend näher erläutert.

Das zunächst ausgeschaltete Laser-Behandlungsgerät kann durch einmaliges kurzes Drücken (kürzer als eine Sekunde) in Betrieb genommen werden. Die Leuchtdiode, die auf die zuletzt eingestellte Behandlungsdauer hinweist, wird mit Dauerlicht angezeigt. Bei einem Neugerät ist dies automatisch die Leuchtdiode, die den Zahlenwerten 2, bzw. 10 der Skalen 11 und 12 zugeordnet ist. Soll die angezeigte Behandlungsdauer verändert werden, so ist der Taster 7 solange zu drücken (länger als 2 Sekunden), bis die Leuchtdiode blinkt. Die Blinkfrequenz beträgt im vorliegenden Fall ca. 2 Hz. Die Behandlungsdauer kann jetzt durch wiederholtes, jeweils kurzes Drücken des Tasters 7 in Ein-Minuten- oder Fünf-Sekunden-Schritten hochgesetzt werden, wobei nach Durchlauf des Leuchtdiodenbandes wieder die erste Leuchtdiode aufleuchtet. Durch längeres Drücken der Taste 7, bis die Leuchtdiode nicht mehr blinkt, sondern Dauerlicht zeigt, lässt sich ein gewünschter Wert speichern. Wird bei einer bestimmten blinkenden Leuchtdiode der Taster länger als 5 Sekunden nicht betätigt, wird diese Leuchtdiode automatisch auf Dauerlicht gesetzt, wodurch der entsprechende Wert vorgewählt und gespeichert ist.

Mit der Steuerung 3 können zudem Zwischenwerte eingestellt werden. Wenn man nun ausgehend von einer bestimmten Leuchtdiode einen anderen Wert einstellen möchte, so muss wiederum die Taste 7 solange gedrückt werden, bis die gerade leuchtende Leuchtdiode blinkt. Durch einmaliges kurzes Drücken der Taste 7 leuchtet neben der gerade blinkenden Leuchtdiode zunächst auch die benachbarte Leuchtdiode auf. Durch Drücken der Taste 7, bis beide Leuchtdioden in Dauerlicht sind, wird dies von der Steuerung so interpretiert, dass hierdurch der Zwischenwert eingestellt wurde, der auf den Skalen jeweils dem Zwischenwert entspricht, der zwischen den beiden Skalenwerten liegt, die wiederum einzelnen Leuchtdioden zugeordnet sind.

Sofern die Behandlungsdauer gerade nicht eingestellt wird, bewirkt ein kurzweiliges Drücken der Taste 7 ein Ausschalten des Geräts.

Durch den Umschalter 8 kann vorgewählt werden, ob es beabsichtigt ist, das Laser-Behandlungsgerät im Akupunkturbetrieb oder aber im Dauerbetrieb zu betreiben. Im Akupunkturbetrieb ist die maßgebliche Skala die Skala 11 mit den Werten von 10 bis 60 Sekunden. Im Bestrahlungsbetrieb ist die maßgebliche Skala die Skala 12 mit Werten von 2 - 12 Minuten. Hat die Bedienperson nun z.B. den Akupunkturbetrieb vorgewählt, so ist als Nächstes ein Akupunkturpunkt zu finden. Hierzu wird das Akupunkturgerät mit der Spitze 6 auf die Haut eines Patienten aufgesetzt oder nahe der Haut angeordnet. Anschließend wird das Akupunkturgerät an der Haut entlang bewegt, wobei der Hautwiderstand gemessen wird. Wenn der Hautwiderstand gering wird, wird ein akustisches Suchsignal ausgegeben. Bei Annäherung an den Akupunkturpunkt kann sich die Frequenz dieses akustischen Signals erhöhen. Die Bedienperson weiß dann, dass sie sich einem Akupunkturpunkt nähert. Auf diese Weise lassen sich Akupunkturpunkte sehr genau feststellen.

Hat man einen Akupunkturpunkt gefunden, so wird die Starttaste betätigt, wodurch die Laserdiode in Betrieb genommen wird. Die Akupunkturbehandlung kann nun beginnen. Nach Ablauf der vorgewählten Behandlungsdauer wird die Laserdiode automatisch abgeschaltet. Um besser erkennen zu können, dass sich das Laser-Behandlungsgerät in Betrieb befindet, werden beim vorliegenden Ausführungsbeispiel beim Betrieb die Leuchtdioden abgeschaltet und alle 15 Sekunden ein Glockensignal ausgegeben. Nach Beendigung der Akupunkturbehandlung wird die Leuchtdiode, die auf die Behandlungsdauer hinweist, wieder angeschaltet.

Mit dem Umschalter 9 kann das Glockensignal auch abgeschaltet werden.

Mit dem neuartigen Laser-Behandlungsgerät lässt sich die Betriebssicherheit deutlich erhöhen und zu lange Behandlungszeiten wirkungsvoll vermeiden.

## Patentansprüche

1. Laser-Behandlungsgerät (1), insbesondere Laser-Akupunkturgerät oder Softlaser, mit einem stiftförmigen Gehäuse (2), einer Steuerungseinrichtung (3), einer Betätigungseinrichtung (7), die zum Bedienen der Steuerungseinrichtung (3) mit dieser verbunden ist und mit einer visuellen Anzeigeeinrichtung (10), die mit der Steuerungseinrichtung (3) zum Darstellen von Information verbunden ist, wobei durch Bedienen der Betätigungseinrichtung (7) ein Zeitraum vorwählbar ist, nach dessen Ablauf die Steuerungseinrichtung (3) das Laser-Behandlungsgerät (1) abschaltet und durch die Anzeigeeinrichtung (10) eine Information darstellbar ist, die dem vorgewählten Zeitraum entspricht **dadurch gekennzeichnet, dass** durch Bedienen der Betätigungseinrichtung (7) das Laser-Behandlungsgerät (1) in Betrieb nehmbar ist

2. Laser-Behandlungsgerät (1) nach Anspruch 1, **dadurch gekennzeichnet, dass** die Betätigungseinrichtung (7) einen vom Benutzer bedienbaren Betätigungsschalter aufweist.

3. Laser-Behandlungsgerät (1) nach Anspruch 2, **dadurch gekennzeichnet, dass** der Betätigungsschalter eine Taste (7) ist.

4. Laser-Behandlungsgerät (1) nach einem der vorangegangenen Ansprüche, **dadurch gekennzeichnet, dass** die Anzeigeeinrichtung mehrere Leuchtdioden (10) aufweist.

5. Laser-Behandlungsgerät (1) nach Anspruch 4, **dadurch gekennzeichnet, dass** die Leuchtdioden (10) in einer Reihe am Gehäuse (2) angeordnet sind.

6. Laser-Behandlungsgerät (1) nach einem der Ansprüche 4 oder 5, **dadurch gekennzeichnet, dass** zumindest sechs Leuchtdioden (10) vorgesehen sind.

7. Laser-Behandlungsgerät (1) nach einem der vorangegangenen Ansprüche, **dadurch gekennzeichnet, dass** den Leuchtdioden (10) benachbart wenigstens eine Skalierung (11, 12) am Gehäuse vorgesehen ist, wobei jeweils Skalenwerte einer Leuchtdiode zugeordnet sind.

8. Laser-Behandlungsgerät (1) nach Anspruch 7, **dadurch gekennzeichnet, dass** die Skalenwerte in einem Bereich von 0 - 60 Sekunden liegen.

9. Laser-Behandlungsgerät (1) nach Anspruch 7, **dadurch gekennzeichnet, dass** die Skalenwerte in einem Bereich von 0 - 12 Minuten liegen.

10. Laser-Behandlungsgerät (1) nach einem der Ansprüche 3 - 9, **dadurch gekennzeichnet, dass** bei Betätigen der Taste (7) zunächst die erste Leuchtdiode aufleuchtet, wobei nach nochmaligem Betätigen der Taste (7) zusätzlich die benachbarte Leuchtdiode aufleuchtet und bei nochmaligem Betätigen nur noch die benachbarte Leuchtdiode aufleuchtet.

11. Laser-Behandlungsgerät (1) nach einem der vorangegangenen Ansprüche, **dadurch gekennzeichnet, dass** eine Sucheinrichtung zum Ermitteln eines Akupunkturpunktes vorgesehen ist, wobei die Sucheinrichtung bei Annäherung an einen Akupunkturpunkt ein akustisches Signal ausgibt.

12. Laser-Behandlungsgerät (1) nach einem der vorangegangenen Ansprüche, **dadurch gekennzeichnet, dass** bei Annäherung an den Akupunkturpunkt die Frequenz des akustischen Signals zunimmt.

13. Laser-Behandlungsgerät (1) nach einem der vorangegangenen Ansprüche, **dadurch gekennzeichnet, dass** das Laser-Behandlungsgerät (1) eine Vorwahleinrichtung (8) aufweist, mit der zwischen einem Akupunkturbetrieb und einem Bestrahlungsbetrieb wählbar ist.

## Claims

1. Laser treatment device (1), in particular a laser acupuncture device or soft laser, with a pen-shaped housing (2), a control unit (3), an operating device (7) which is connected to the control unit (3) in order to operate the latter, and a visual display unit (10) connected to the control unit (3) for displaying information, and the operating device (7) can be operated in order to pre-select a period, after the elapse of which the control unit (3) switches off the laser treatment device (1), and information corresponding to the pre-selected period can be displayed by the display unit (10), **characterised in that** when the operating device (7) is operated, the laser treatment device (1) can be placed in operating mode.

2. Laser treatment device (1) as claimed in claim 1, **characterised in that** the operating device (7) has an operating switch which can be operated by the user.

3. Laser treatment device (1) as claimed in claim 2, **characterised in that** the operating switch is a push-button (7).

4. Laser treatment device (1) as claimed in one of the preceding claims, **characterised in that** the display unit has several light-emitting diodes (10).

5. Laser treatment device (1) as claimed in claim 4, **characterised in that** the light-emitting diodes (10) are disposed in a row on the housing (2).

6. Laser treatment device (1) as claimed in one of claims 4 or 5, **characterised in that** at least six light-emitting diodes (10) are provided.

7. Laser treatment device (1) as claimed in one of the preceding claims, **characterised in that** at least one scale (11, 12) is provided on the housing adjacent to the light-emitting diodes (10) and scale values are respectively assigned to a light-emitting diode.

8. Laser treatment device (1) as claimed in claim 7, **characterised in that** the scale values lie within a range of 0 - 60 seconds.

9. Laser treatment device (1) as claimed in claim 7, **characterised in that** the scale values lie within a range of 0 - 12 minutes.

10. Laser treatment device (1) as claimed in one of claims 3 - 9, **characterised in that** when the push-button (7) is operated, the first light-emitting diode lights up in the first instance, and when the push-button (7) is operated again, the adjacent light-emitting diode lights up in addition, and when operated again, only the adjacent light-emitting diode lights up.

11. Laser treatment device (1) as claimed in one of the preceding claims, **characterised in that** a searching device for determining an acupuncture point is provided, which searching device emits an acoustic signal as it approaches an acupuncture point.

12. Laser treatment device (1) as claimed in one of the preceding claims, **characterised in that** on approaching the acupuncture point, the frequency of the acoustic signal increases.

13. Laser treatment device (1) as claimed in one of the preceding claims, **characterised in that** the laser treatment device (1) has a pre-selection device (8), by means of which a selection can be made between an acupuncture mode and a radiation mode.

## Revendications

1. Appareil de traitement au laser (1), en particulier appareil d'acupuncture au laser ou laser doux, comprenant un boîtier en forme de crayon (2), un dispositif de commande (3), un dispositif d'actionnement (7) qui est relié au dispositif de commande (3) pour actionner celui-ci, et un indicateur visuel (10) qui est relié au dispositif de commande (3) pour présenter des informations, étant donné que grâce à la manoeuvre du dispositif d'actionnement (7), on peut présélectionner un laps de temps après l'écoulement duquel le dispositif de commande (3) arrête l'appareil de traitement au laser (1), et que l'indicateur (10) permet de présenter une information qui correspond au laps de temps présélectionné, **caractérisé en ce que** grâce à la manoeuvre du dispositif d'actionnement (7), l'appareil de traitement au laser (1) peut être mis en marche.

2. Appareil de traitement au laser (1) selon la revendication 1, **caractérisé en ce que** le dispositif d'actionnement (7) comporte un commutateur d'actionnement apte à être manoeuvré par l'utilisateur.

3. Appareil de traitement au laser (1) selon la revendication 2, **caractérisé en ce que** le commutateur d'actionnement est constitué par un poussoir (7).

4. Appareil de traitement au laser (1) selon l'une des revendications précédentes, **caractérisé en ce que** l'indicateur comporte plusieurs diodes lumineuses (10).

5. Appareil de traitement au laser (1) selon la revendication 4, **caractérisé en ce que** les diodes lumineuses (10) sont disposées sur une rangée sur le boîtier (2).

6. Appareil de traitement au laser (1) selon la revendication 4 ou 5, **caractérisé en ce qu'**il est prévu au moins six diodes lumineuses (10).

7. Appareil de traitement au laser (1) selon l'une des revendications précédentes, **caractérisé en ce qu'**il est prévu près des diodes lumineuses (10) au moins une graduation (11, 12) sur le boîtier, une valeur de graduation étant associée à chacune de ces diodes.

8. Appareil de traitement au laser (1) selon la revendication 7, **caractérisé en ce que** les valeurs de graduation sont situées dans une plage de 0-60 secondes.

9. Appareil de traitement au laser (1) selon la revendication 7, **caractérisé en ce que** les valeurs de graduation sont situées dans une plage de 0-12 minutes.

10. Appareil de traitement au laser (1) selon l'une des revendications 3 à 9, **caractérisé en ce que** lors de l'actionnement du poussoir (7), la première diode lumineuse s'allume tout d'abord, étant précisé qu'après un nouvel actionnement du poussoir (7), la diode voisine s'allume en supplément et que lors d'un nouvel actionnement, il n'y a plus que la diode voisine qui est allumée.

11. Appareil de traitement au laser (1) selon l'une des revendications précédentes, **caractérisé en ce qu'**il est prévu un dispositif de recherche pour rechercher un point d'acupuncture, ce dispositif de recherche émettant un signal acoustique quand on approche d'un point d'acupuncture.

12. Appareil de traitement au laser (1) selon l'une des revendications précédentes, **caractérisé en ce que** lorsqu'on approche du point d'acupuncture, la fréquence du signal acoustique augmente.

13. Appareil de traitement au laser (1) selon l'une des revendications précédentes, **caractérisé en ce qu'**il comporte un dispositif de présélection (8) grâce auquel on peut choisir entre un mode d'acupuncture et un mode d'irradiation.
